# EUROPEAN PATENT APPLICATION

(11) **EP 2 523 001 A1**
(43) Date of publication of application: **14.11.2012**
(21) Application number: 10842119.9
(22) Date of filing: 07.10.2010
(51) Int. Cl.: G01N 33/531, G01N 33/543

(54) **REAGENT COMPOSITION FOR IMMUNOCHROMATOGRAPHY**

(30) Priority: 08.01.2010 JP 2010003421
(71) Applicant: Tanaka Kikinzoku Kogyo K.K., Chiyoda-ku Tokyo 100-6422 (JP)
(72) Inventor: ITOH Daisuke, Hiratsuka-shi Kanagawa 254-0076 (JP); SHIBAI Yusuke, Hiratsuka-shi Kanagawa 254-0076 (JP)
(74) Representative: Peaucelle, Chantal
(86) International application number: PCT/JP2010/067617
(87) International publication number: WO 2011/083604

(57) **Abstract**

The invention relates to a reagent composition, or a sample thinner, for immunochromatography which are more effective in suppressing nonspecific reactions than the conventional counterparts, which use an immunochromatography device to detect a target substance; it also relates to a high performance and highly sensitive immunochromatography device and a detective kit capable of prompt and simple detection work.

In detecting a target substance in a sample by means of immunochromatography method, it is possible to suppress nonspecific reactions through a use of a reagent composition for immunochromatography or a development solution for immunochromatography or a sample thinner for immunochromatography which contain a buffer solution, a chelating agent, and a non-ionic surfactant.

## Description

The present invention relates to a high performance and highly responsive reagent composition for immunochromatography or a developing solution for immunochromatography, which are effective in suppressing nonspecific reactions. Furthermore, the present invention relates to a detective device and a detection method, which can detect and measure an object for detection in an analyzed sample promptly and simply through suppression of nonspecific reactions. In particular, the present invention relates to a detective device which is capable of a prompt, simple and high precision detection through supplying of a detection reagent directly to a sample introduction part of the detective device without going through a pretreatment of the analyzed sample.

In recent years, increased is the importance of an immunoassay of strip type for immunochromatography, which does not require pretreatment of the analyzed sample, as a simple in vitro diagnostic kit or a portable diagnostic system which detects an antigen in a sample liquid based on the nonspecific reactivity of the antibody. In particular, a pregnancy test kit is a familiar immunochromatography kit sold as a non-prescription pharmaceutical commodity. A research has been made on an immunochromatography device which can detect pregnancy by making use of an antibody which undergoes a nonspecific reaction with hCG in urine, as hCG, which is an excretion from the placenta, is excreted into the urine of a pregnant woman.

Now, if the analyzed sample is urine, various components are included in it, and composition and specific gravity differ from one urine to another so that they affect the immunochemical bonding quality in no small way, thereby becoming causes to lessen the precision in the result obtained from the assay method which makes use of immunochemical bonding. In order to minimize the effects of these obstructive causes, there have been countermeasures making use of various supportive means (ref. IP Publication 1).

Also, there is known a technology wherein a chelating agent such as ethylenediaminetetraacetic acid (hereinafter merely "EDTA").2Na, EDTA.3K, and diethylene triamine pentaacetic acid (hereinafter merely "DTPA") is added in order to prevent the denaturation of hemoglobin at the time when hemoglobin in an analyzed sample is to be measured by means of high performance liquid chromatography (see IP Publication 2).

However, it has been confirmed that EDTA cannot be expected to have sufficient ability in stabilizing the hemoglobin in analyzed samples of feces, or the like, and it is known that a better stabilization effect is obtained if a water-soluble metallic complex of a transition metal ion is used rather than EDTA alone (ref. IP Publication 3).

Furthermore, there is known a method wherein at the time of the measurement of hemoglobin in the analyzed sample in the course of immunochromatography, a substance consisting of a metal ion selected from iron ion, copper ion and lead ion and of a chelating agent is added as a stabilizer into a sensitizing metal colloidal reagent solution (ref. IP Publication 4).

On the other hand, with regard to immunochromatography device, it has been a conventionally recognized problem that the coloring of a background (coloring of the decision part excluding the immobile phase antibody) and the blank color generation (color generation of immobile phase in a case where a target substance is absent) not only decrease the SN ratio but also cause malfunctioning. The background coloration is said to be caused by a hydrophobic bonding between a visualized mobile phase antibody and a porous carrier, and the blank color generation is said to be caused by an electrical interaction between a mobile phase antibody having a negative electric charge and an immobile phase carrier having a positive electric charge. As a countermeasure, a immunochromatography device is disclosed wherein an additive immersion part is provided, in a porous carrier which constitutes a test piece, at a location between a sample introduction part and a decision part, and at least one of substances capable of canceling such hydrophobic bonding and electrical interaction, for example a surfactant, an ammonium salt, and a pH buffer, is let to be carried in it (ref. IP Publication 5).

Also, in a immunochromatography detection method which makes use of a developing solution such as pH buffer, various additives have been used for suppression of side reactions arising from biological affinity or for suppression of nonspecific reactions, and examples of such additives for promotion of antigen-antibody reaction or for suppression of nonspecific reactions include proteins (e.g., bovine serum albumin, casein, gelatin, etc.), high molecular compounds (e.g., polyethylene glycol, dextran, methyl cellulose, polyvinylpyrrolidone, etc.), nonionic surfactants (e.g., Tween 20, Triton X-100, etc.), ionic surfactants or polyanions (e.g., dextran sulfate, heparin, polystyrene sulfonic acid, hyaluronic acid, chondroitin sulfate, etc.) or their salts (IP Publication 6).

However, although nonionic surfactants (e.g., Tween 20, Triton X-100, etc.), ionic surfactants and polyamines are mentioned as surfactants used as means for preventing the coloring of the background or the blank color generation or for suppression of side reactions arising from biological affinity or for suppression of nonspecific reactions, the fact remains that there has not yet been a satisfactory result obtained with any of these to solve the problem of the nonspecific reaction.

The inventors of the present invention specially singled out nonionic surfactants out of nonionic surfactants (e.g., Tween 20, Triton X-100, etc.), ionic surfactants and polyamines, and conducted experiments on them and as the result they found that nonspecific reactions still occur in spite of them and that the suppression of the nonionic reactions is not sufficient.
IP Publication 1: Japanese Translation of PCT International Application No. 2007-526443
IP Publication 2: Japanese Published Patent Application No. H02(1990)-221859
IP Publication 3: Japanese Published Patent Application No. H07(1995)-229902
IP Publication 4: Japanese Published Patent Application No. 2000-146967
IP Publication 5: Japanese Published Patent Application No. H11(1999)-153601
IP Publication 6: Japanese Published Patent Application No. 2007-322310

It is an object of the present invention to provide a high performance and highly responsive reagent composition for immunochromatography or a developing solution for immunochromatography, which are more effective in suppressing nonspecific reactions than the conventional arts. It is also an object of the present invention to provide an immunochromatography device, which can detect and measure an object for detection more promptly, simply and accurately through suppression of nonspecific reactions than the conventional devices can. For example, the invention proposes an immunochromatography device which enables a prompt and simple pregnancy detection test by means of a specific reaction with human chorionic gonadotropin (hCG) in the urine.

It is also an object of the present invention to provide a detective device which is capable of a prompt, simple and high precision detection through supplying of a detection reagent directly to a sample introduction part of the detective device thereby suppressing nonspecific reactions and causing a specific reaction with the object for detection in the analyzed sample, without going through a pretreatment of the analyzed sample. For example, the invention provides a detective kit which enables a prompt, easy and highly accurate pregnancy detection by supplying the urine directly to the sample introduction part of this detective device.

The present invention also relates to a method for detecting an object of detection in the analyzed sample by suppressing the nonspecific reactions based on the immunochromatography method.

The present invention provides reagent compositions (a) through (j), described herein below, for immunochromatography, developing solution for immunochromatography, an immunochromatography device and an immunochromatography method which make use of them, and a detective kit.
(a) A first feature of the present invention lies in a reagent composition for immunochromatography for detecting, in an analyzed sample, a target object selected from a group consisting of human chorionic gonadotropin (hCG), luteinizing hormone (LH), follicle-stimulating hormone (FSH) and thyroid stimulating hormone (TSH), the reagent composition being characterized by containing a buffer solution, a chelating agent, and a non-ionic surfactant made of a polyoxyethylene/polyoxypropylene block copolymer for which the molar ratio of the oxyethylene repetition units in the polyoxyethylene block having an alkyl group at one end to the oxypropylene repetition units in the polyoxypropylene block is in a range of 0.1 - 1.5.
(b) A second feature of the present invention lies in a reagent composition as described in (b) above, in which the chelating agent is based on aminocarboxylic acid.
(c) A third feature of the present invention lies in a diluted reagent composition for immunochromatography for detecting, in an analyzed sample, a target object selected from a group consisting of human chorionic gonadotropin (hCG), luteinizing hormone (LH), follicle-stimulating hormone (FSH) and thyroid stimulating hormone (TSH), the reagent composition being characterized by containing a buffer solution, a chelating agent, and a non-ionic surfactant made of a polyoxyethylene/polyoxypropylene block copolymer for which the molar ratio of the oxyethylene repetition units in the polyoxyethylene block having an alkyl group at one end to the oxypropylene repetition units in the polyoxypropylene block is in a range of 0.1 - 1.5.
(d) A fourth feature of the present invention lies in an immunochromatography device characterized by: comprising substantially and sequentially of a sample introduction part, a marker holding part, a chromatography medium part, a detection part for detecting from an analyzed sample a target object selected from a group consisting of human chorionic gonadotropin (hCG), luteinizing hormone (LH), follicle-stimulating hormone (FSH) and thyroid stimulating hormone (TSH), and an absorption part; and also by having, at a location between an end of the sample introduction part and the absorption part, a site which includes a reagent composition containing a buffer solution, a chelating agent, and a non-ionic surfactant made of a polyoxyethylene/polyoxypropylene block copolymer for which the molar ratio of the oxyethylene repetition units in the polyoxyethylene block having an alkyl group at one end to the oxypropylene repetition units in the polyoxypropylene block is in a range of 0.1 - 1.5.
(e) A fifth feature of the present invention lies in an immunochromatography device as described in (d) above, in which the chelating agent is based on aminocarboxylic acid.
(f) A sixth feature of the present invention lies in a detective kit characterized by including an immunochromatography device, which comprises substantially and sequentially of a sample introduction part, a marker holding part, a chromatography medium part, a detection part for detecting in an analyzed sample a target object selected from a group consisting of human chorionic gonadotropin (hCG), luteinizing hormone (LH), follicle-stimulating hormone (FSH) and thyroid stimulating hormone (TSH), and an absorption part; and also (the device) has, at a location between an end of the sample introduction part and the absorption part, a site which includes a buffer solution, a chelating agent, and a non-ionic surfactant made of a polyoxyethylene/polyoxypropylene block copolymer for which the molar ratio of the oxyethylene repetition units in the polyoxyethylene block having an alkyl group at one end to the oxypropylene repetition units in the polyoxypropylene block is in a range of 0.1 - 1.5.
(g) A seventh feature of the present invention lies in a developing solution for immunochromatography used for detecting in an analyzed sample a target object selected from a group consisting of human chorionic gonadotropin (hCG), luteinizing hormone (LH), follicle-stimulating hormone (FSH) and thyroid stimulating hormone (TSH); the developing solution being characterized by containing a buffer solution, a chelating agent, and a non-ionic surfactant made of a polyoxyethylene/polyoxypropylene block copolymer for which the molar ratio of the oxyethylene repetition units in the polyoxyethylene block having an alkyl group at one end to the oxypropylene repetition units in the polyoxypropylene block is in a range of 0.1 - 1.5.
(h) An eighth feature of the present invention lies in an immunochromatography method for detecting in an analyzed sample a target object selected from a group consisting of human chorionic gonadotropin (hCG), luteinizing hormone (LH), follicle-stimulating hormone (FSH) and thyroid stimulating hormone (TSH); the detection method being characterized by a use, as a developing solution constituting a mobile phase, of a developing solution for immunochromatography containing a buffer solution, a chelating agent, and a non-ionic surfactant made of a polyoxyethylene/polyoxypropylene block copolymer for which the molar ratio of the oxyethylene repetition units in the polyoxyethylene block having an alkyl group at one end to the oxypropylene repetition units in the polyoxypropylene block is in a range of 0.1 - 1.5.

By virtue of its inclusion of a buffer solution, a chelating agent, and a non-ionic surfactant, the reagent composition for immunochromatography of the present invention is capable of suppressing nonspecific reactions for some unknown suppressing mechanism (the details of which are yet to be discovered), during the detection work for the target object (antigen) existing in the analyzed sample, so that the sensitivity of the detection performance is not dulled and it is possible to obtain a precision result. For example, on account of the fact that the nonspecific reactions are profoundly restricted, the detection of the hCG in the urine as the sample will have an unmitigated sensitivity and as a result an accurate judgment of pregnancy test result is obtainable.

Also, to have, in a part of the immunochromatography device, a site which includes a reagent composition for immunochromatography of the present invention means that the reagent composition for immunochromatography is applied to, absorbed or immersed in a region between an end of the sample introduction part and the absorption part, and thereafter the composition is dried to thereby be supported, held or retained. for example, in the case of a dried immunochromatography device, after application or immersion of the reagent composition to a sample pad (sample introduction part) of the immunochromatography device, it is not necessary to conduct a pre-treatment on the urine sample, in the course of a detection of hCG from the urine sample, so that, characteristically, it is possible to add the urine directly upon the sample introduction part of the detective kit whereby the nonspecific reactions are suppressed and the specific reaction with the hCG in the urine takes place and thus a prompt and simple pregnancy test is enabled. In having the site which includes the reagent composition for immunochromatography of the present invention provided at a position in the immunochromatography device, that is, in having it supported or held or retained, such a position may be arbitrarily selected so long as it is in the region between an end of the sample introduction part and the absorption part. For example, it may be the sample introduction part, the marker holding part, or a position on the immunochromatography medium part that is closer to the sample introduction part than the detection part; but the location, size (width), concentration, etc. of the reagent composition application may be determined arbitrarily based on the consideration of the efficiency of the composition, and this is within the scope of design choice.

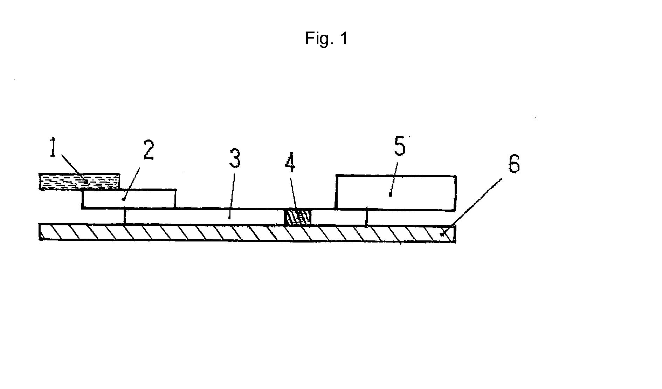

Fig. 1 a schematic chart of a test piece of immunochromatography device Representation of reference numerals
- 1:: sample introduction part (sample pad)
- 2:: labeled material retained part
- 3:: chromatography medium
- 4:: detection part
- 5:: absorption part
- 6:: backing sheet

Now, the present invention will be described in detail.

The inventors of the present invention zealously repeated research work on immunochromatography device, and, as a result, found that through a use, as the reagent composition for immunochromatography, of a reagent composition containing a buffer solution, a chelating agent, and a non-ionic surfactant, it is possible to undo to a high degree the hydrophobic bonding between the mobile phase antibody and the porous carrier and the electrical interaction between the mobile phase antibody and the immobile phase carrier by the help of an interplay among the buffer solution, the chelating agent, and the non-ionic surfactant, and that it is also possible to stabilize the detection target substance and the labeled detection agent, and to stabilize the complex which is a product of a reaction between the detection target substance and the labeled antibody by virtue of a synergism between the buffer solution, the chelating agent, and the non-ionic surfactant, and at the same time it was first recognized that it is possible to suppress very profoundly the nonspecific reactions and to detect/measure the detection target substance in the analyzed sample specifically with high sensitivity and speediness through effecting of a function by which their movement as the mobile phases is facilitated, and thus the invention was completed.

As the buffer solution, which constitutes one of the components of the reagent composition for immunochromatography of the present invention, there is no special limitation to it so long as it has a buffering ability which is not substantially mitigated as its concentration changes with the addition of sample, evaporation or dilution of the sample or as it encounters more or less of a foreign matter which mixes in from the outer environment.

Examples of the buffer solution useful in the present invention include: acetate buffer solution (acetic acid + sodium acetate), phosphate buffer solution (phosphoric acid + sodium phosphate), citrate buffer solution (citric acid + sodium citrate), borate buffer solution, tris-HCl buffer solution (tris(hydroxyl methyl)aminomethane + hydrochloric acid), TE buffer solution (tris + ethylenediaminetetraacetic acid), TAE buffer solution (tris + acetatic acid + ethylenediaminetetraacetic acid), TBE buffer solution (tris + boric acid + ethylenediaminetetraacetic acid) and HEPES buffer solution (2-[4-(2-hydroxyl ethyl)-1-piperazinyl]ethane sulfonic acid). Preferable choices include acetate buffer solution, phosphate buffer solution and tris-HCl buffer solution, and a more preferred choice is tris-HCl buffer solution.

As for the chelating agent, which constitutes one of the components of the reagent composition for immunochromatography of the present invention, there is no special limitation to it so long as it has a capacity of becoming a ligand having a plurality of coordination positions.

Examples of the chelating agent useful in the present invention include: ethylenediamine, dipyridine, ethylenediaminetetraacetic acid (hereinafter merely "EDTA"), EDTA·2Na, EDTA·3Na, EDTA·4Na, EDTA derivative (for example, EDTA·2NH₄, EDTA·3K, and EDTA·special amine salt), EDTA metal salt (for example, EDTA·Ca·2Na), and chelating agents of hydroxyethhyl ethylenediamine triacetic acid (HEDTA) type, dihydroxyethhyl ethylenediamine diacetic acid (DHEDDA) type, 1,3-propanediamine tetraacetic acid (1,3PDTA) type, diethylenetriamine pentaacetic acid (DTPA) type, triethylenetetraamine hexaacetic acid (TTHA) type, nitrilotriacetic acid (NTA) type, gluconic acid type, hydroxyethyl imino diacetic acid (HIMDA) type, L-asparagine acid-N,N-diacetic acid (ASDA) type, aminotrimethylene phosphonic acid (NTMP) type, hydroxyethane phosphonic acid (HEDP) type, and 3-hydroxy-2,2'-iminodisuccinic acid-4 sodium, phenanthroline, porphyrin, and crown ether.

A preferable chelating agent of the present invention is aminocarboxylic acid chelating agent.

There is no specific preference in selecting an aminocarboxylic acid chelating agent so long as the selected one is a chemical compound having an amino group and a carboxylic acid functional group which are capable of undergoing a complexation with heavy metal ion and alkaline earth-metal ion. Examples include the above-mentioned ethylenediaminetetraacetic acid (EDTA), nitrilotriacetic acid (NTA), diethylenetriamine pentaacetic acid (DTPA), and hydroxyethhyl ethylenediamine triacetic acid (HEDTA). More recommended is to use ethylenediaminetetraacetic acid (EDTA).

For the non-ionic surfactant, which constitutes one of the components of the reagent composition for immunochromatography of the present invention, some of the examples are polyoxyethylene alkyl ether, polyoxyethylene/polyoxypropylene alkyl ether, polyoxyethylene sorbitan fatty acid ester (of "Tween" series, a proprietary name of product group), polyoxyethylene p-t-octyl phenyl ether (of "Triton" series, a proprietary name of product group), polyoxyethylene p-t-nonyl phenyl ether (of "TritonN" series, a proprietary name of product group), alkyl polyglucoside, fatty acid diethanol amid, and alkyl monoglyceryl ether. The non-ionic surfactant may consist of any single one of these or a mixture of two or more of these.

A more suitable example of the non-ionic surfactant, which constitutes one of the components of the reagent composition for immunochromatography of the present invention, is polyoxyethylene/polyoxypropylene alkyl ether. In more particular terms, it is a polyoxyethylene/polyoxypropylene block copolymer having an alkyl group at one of its two terminals, and this alkyl group at the one terminal may be in the form of a linear or a branched chain, and preferably the alkyl group has 1 - 18 carbon atoms and more preferably 10 - 18 carbon atoms. In the case of a polyoxyethylene/polyoxypropylene block copolymer having a hydroxyl group at both of its two terminals, the denaturation of the impurities such as glycoprotein, which causes nonspecific reactions, is thwarted, the solubility and dispersability with respect to the treatment liquid are insufficient, or the affinity to the labeled antibody and the detection target antibody by virtues of electrostatic or hydrophobic bonding is not controllable wherefore the nonspecific reactions are not contolled and hence an accurate decision is not made. In the case of a copolymer having one alkyl group with more than 18 carbon atoms, the making of the complex which is a product of a reaction between the detection target substance and the labeled antibody and which is indispensable for the measurement, is prevented, so that a sufficient sensitivity is not obtained for the decision. Furthermore, the molar ratio of the oxyethylene repetition units in the polyoxyethylene block to the oxypropylene repetition units in the polyoxypropylene block is preferably in a range of 0.1 - 1.5, and more preferably 0.15 - 1.1. The alkyl group at the one terminal is preferably bonded to the polyoxyethylene block. If the above-mentioned molar ratio of the repetition units is less than 0.1, the denaturation of the impurities such as glycoprotein, which causes nonspecific reactions, is thwarted, the solubility and dispersability with respect to the treatment liquid are insufficient, or the affinity to the labeled antibody and the detection target antibody by virtues of electrostatic or hydrophobic bonding is not controllable wherefore the nonspecific reactions are not controlled and hence an accurate decision is not made. If it exceeds 1.5, the making of the complex which is a product of a reaction between the detection target substance and the labeled antibody and is indispensable for the measurement, is prevented, so that a sufficient sensitivity is not obtained for the decision.

The content of the non-ionic surfactant, which constitutes one of the components of the reagent composition for immunochromatography of the present invention, may account for 0.01 - 10 weight % of the reagent composition for immunochromatography, and preferably 0.05 - 5 weight %. If it is less than 0.01 weight %, the nonspecific reactions are not suppressed and thus a precision judgment is not possible. If it is more than 10 weight %, while there is no further gain in terms of the suppression of the nonspecific reactions, the extra wasteful addition of the surfactant can weaken the economy.

As f the non-ionic surfactant, which constitutes one of the components of the reagent composition for immunochromatography of the present invention, it is desirable to use only a polyoxyethylene/polyoxypropylene block copolymer having an alkyl group at one of its terminals. However, so long as no adversary consequence takes place, it is permissible to add some other non-ionic surfactant(s) and/or ionic surfactant(s).

The concentration of the buffer solution, which constitutes one of the components of the reagent composition for immunochromatography of the present invention, is in a range of 0.01 - 250 mM, preferably in a range of 10 - 200 mM, and more preferably 30 - 180 mM. If the concentration is lower than 0.01 mM, the buffering effect is insufficient. If higher than 250 mM, the unnecessary surplus is nothing but a waste and non-economical.

It is desirable that the reagent composition for immunochromatography of the present invention contains no other buffer agent but tris-HCl buffer. However, so long as no adversary consequence takes place, it is permissible to add some other buffer(s).

The concentration of the chelating agent, which constitutes one of the components of the reagent composition for immunochromatography of the present invention, is in a range of 0.01 - 10 mM, preferably in a range of 0.1 - 5 mM, and more preferably 0.5 - 2 mM. If the concentration is lower than 0.01 mM, the nonspecific reactions are not suppressed and thus a precision judgment is not possible. If it is more than 10 mM, the unnecessary surplus is nothing but a waste and non-economical.

It is permissible for the reagent composition for immunochromatography of the present invention to contain one or more of additives which are known to suppress a secondary reaction based on biological affinity and/or a nonspecific reaction; examples of such additives are proteins (e.g., bovine serum albumin, casein, gelatin, etc.), high molecular compounds (e.g., polyethylene glycol, methyl cellulose, polyvinylpyrrolidone, polyvinyl alcohol, dextran, etc.), ionic surfactants or polyanions (e.g., dextran sulfate, heparin, polystyrene sulfonic acid, chondroitin sulfate, etc.) or an antimicrobial agent, which are capable of promoting antigen-antibody reaction or suppressing nonspecific reactions; use of such additives can be effective and not prohibited. It is also possible and effective and not prohibited to install one or more of the proteins, the high molecular compounds, the ionic surfactants or the polyanions, or the antimicrobial agent which are capable of promoting antigen-antibody reaction or suppressing nonspecific reactions, at a migration pathway on the chromatography medium.

As for a method for creating a position which includes the ingredients of the reagent composition for immunochromatography of the present invention, it is possible to adopt one wherein, for example, the reagent composition for immunochromatography of the present invention is applied or immersed in a sample pad (sample introduction part) of the immunochromatography device, and is dried, whereby the reagent composition is captured or held. Another embodiment for holding the reagent composition for immunochromatography of the present invention on the immunochromatography medium is to provide an additive agent holding part at an arbitrary position between an end of the sample introduction part and the absorption part, and let the reagent held there. For example, the position for the installation can be on the sample introduction part, labeled material (marker) holding part or immunochromatography medium. It is preferable that the installation or the holding is done only at the sample introduction part and/or the labeled material retained part.

The method for using the reagent composition for immunochromatography of the present invention is not limited to what are described above, but the reagent composition may be used as a developing solution or a thinner fluid for a sample. In the case of using as a developing solution, water is usually used as the solvent, and to this are added a buffer solution, a chelating agent and a non-ionic surfactant. There is no specified order for the addition of these, and it is permissible to add them at once. In the case of using the reagent composition as the developing solution, it is possible to cause the development by supplying and dripping on the sample pad (sample introduction part) a mixture preliminarily prepared of the detection sample and the developing solution, or it is possible to cause the development by supplying and dripping the sample on the sample pad (sample introduction part) and then by supplying and dripping the developing solution on the sample pad (sample introduction part). In the case of using the reagent composition as the sample thinner, the diluted sample wherein the sample has been thinned may be used as the development solution, as it is, and the diluted sample is supplied and dripped on the sample pad (sample introduction part).

Some of the chief examples of the sample (object to be examined), which contains the target substance for detection, used in the present invention, are biological specimen such as blood, serum, blood plasma, urine, saliva, cerebrospinal fluid, sweat, tear, amniotic fluid, nipple discharge, nasal mucus, phlegm, rinse fluid from nasal cavity or pharynx, exudates from skin, fluid extract from tissue, cells, and feces.

The target substance for detection meant in connection with the present invention is not particularly limited, but can be matters which have or produce an element that specifically couples with it, for example, by a reaction between an antigen and an antibody. The target substance for detection can be something that itself has immunogenicity, like complete antigen, or it can be something that itself does not have immunogenicity itself, like hapten (incomplete antigen), but has an ability to acquire immunogenicity through a chemical change of itself. Anything that has or creates an element that specifically couples with any of such target substances for detection will do and pass as a monoclonal antibody or a polyclonal antibody. Some of the examples of the target substance for detection applicable to the present invention are peptide hormone (growth hormone (GH), adrenocorticotropic hormone (ACTH), melamine cell stimulating hormone (MSH), prolactin, thyroid stimulating hormone (TSH), luteinizing hormone (LH), follicle-stimulating hormone (FSH), pituitary hormone, calcium metabolism regulation hormone, pancreatic hormone, gastrointestinal hormone, vasoactivity hormone, a placental hormone such as human chorionic gonadotropin, prostatic acid phosphatase (PAP), prostata specific antigen (PSA), alkaline phosphatase, transaminase, trypsin, pepsinogen, alpha-fetoprotein (AFP), a cancer specific substance such as carcinoembryonic antigen (CEA), a serum protein component such as immunoglobulin G (IgG), rheumatoid factor, urokinase, ferritin, substan P, an estrogen such as estrone, fecal occult blood, syphilis antibody, influenza virus, adenovirus, rotavirus, HBs antigen, anti-hepatitis B surface antigen, chlamydial antigen, group A beta streptococcal antigen, natural or synthetic corpus luteum hormone such as progesterone, male hormone such as testosterone, adrenocortical hormone such as cortisol, cholesterol, bile acid, cardiotonic steroid, other steroids such as sapogenin, epinephrine, dopamine, bioactive alkaloids, amino group-containing psychotropic drugs, small peptides such as TRH, thyroid hormones such as diiodothyronine, prostaglandins, vitamin group, antibiotics such as penicillin, other in-vivo elements, in-vivo dosed medicine, and its metabolic products. Preferable examples of target substance for detection are human chorionic gonadotropin (hCG), luteinizing hormone (LH), follicle-stimulating hormone (FSH) and thyroid stimulating hormone (TSH), and hCG is the most preferable.

In the case where urine is used as the analyzed sample in the present invention, it only suffices to supply/drip the urine onto the sample pad in the immunochromatography device, for urine requires no pretreatment. Hence, the pregnancy test becomes so simple that it is possible to conduct it by anyone at any place, such as in home. In this case, the detection target substance in the analyzed sample is the hCG which is secreted from the pregnant woman's placenta and discharged into the urine. The reagent composition for immunochromatography of the present invention is especially effective for urine sample, and provides a result of detection of the target substance with higher sensitivity, as it suppresses nonspecific reactions involving the impurities in the urine.

The immunochromatography device for the detection of the hCG which is secreted into the urine has a known structure and is based on a known method of performance and detection.

It is possible to have the reagent composition for immunochromatography of the present invention held in the sample pad of a conventional immunochromatography device - this is done, for example, by immersing the pad with the composition, and drying it; then this immunochromatography device is used to identify and measure the target substance in the analyzed sample, for example hCG in the urine, by virtue of the specific reactions such as a reaction between the antigen and the antibody.

The sample introduction part (1) in Fig. 1 is constituted by a porous sheet, which has a characteristic such that it absorbs a sample promptly but can hold it so weakly that the sample can migrate to the reaction part speedily. The porous sheet can be cellulose filter paper, glass filter paper, polyurethane, polyacetate, cellulose acetate, nylon, cotton, etc. A preferred porous sheet for the present invention is glass filter paper. In the present invention in order to suppress the nonspecific reactions, it is possible, for example, to immerse the reagent composition for immunochromatography, which includes buffering solution, chelating agent and nonionic surfactant, into the sample introduction pad (1) beforehand, and then to dry it whereby the holding of the composition is done. For example, it is possible to adopt an embodiment wherein any of the elements of the reagent composition for immunochromatography, such as tris-HCl buffer solution, EDTA, and polyoxyethylene block having an alkyl group at one end, is set to be retained or held beforehand at the sample pad.

In the labeled material retained part (2), a labeled detection agent, which labels a reagent ingredient by means of a labeling ingredient, is held or retained. The labeling ingredient can be a metal colloidal particle such as gold colloidal particle and silver colloidal particle, a colored latex particle obtained through coloring of synthetic high polymer produced by (co-)polymerization of various monomers, an enzyme, a fluorescence chemical compound, etc. The reagent ingredient is a particle or a molecule capable of perceiving an analyzed substance, and preferably it is a monoclonal antibody or a polyclonal antibody or even a fragment of such antibody (a second reagent).

The chromatography medium (3) consists of a film retainer and a detection part (4) formed on the retainer. The retainer can be made of any material so long as the material is capable of absorbing a tested sample by means of capillary phenomenon and of letting it migrate. For example, one may select from cellulose nitrate, cellulose acetate, nylon, polyether sulfone, polyvinyl alcohol, polyester, glass fiber, polyolefin, cellulose, and a synthetic polymer made of a mixture of any of these fibers. In the detection part (4), a monoclonal antibody or a polyclonal antibody or a fragment of such antibody (a first reagent) is fixedly retained on a cellulose nitrate sheet.

To make the absorption part (5), a material capable of promptly absorbing an excessive amount of sample or glass filtration paper is used.

The backing sheet (6) forms the base structure. By applying an adhesive paste or an adhesive tape to one side of it, that side is made adhesive, and to this adhesive side all or part of the sample introduction part (1), the labeled material retained part (2), the chromatography medium (3), the detection part (4), and the absorption part (5) are attached, each one being close to another. The backing sheet (6) can be made of any material so long as the material with the adhesive does not pass the sample liquid through it.

One or both of the reagent ingredient (the first reagent) used in the detection part (4) and the reagent ingredient (the second reagent) used for the labeling reagent can be a monoclonal antibody or a polyclonal antibody, but it is preferable that at least one of them is a polyclonal antibody if the manufacturing cost and the stable supply of the antibody are considered without sacrificing the specificity.

Furthermore, the latter reagent ingredient (the second reagent) used as the labeling reagent is more preferably a monoclonal antibody having high specificity from the viewpoint of measurement sensitivity and the like, and the former reagent ingredient (the first reagent) used as the detection part (4) is more preferably a polyclonal antibody.

Monoclonal antibodies and polyclonal antibodies and their fragments are in the public domain, and are available, and are able to be adjusted in known procedures. Examples of antibody production animals are human, mouse, rat, rabbit, goat, etc. The immunoglobulin G can b any of IgG, IgM, IgA, IgE, and IgD.

Monoclonal antibody is obtained in the usual method, that is, hybridizing mouse's spleen cell with its myeloma cell, which cells have been immunized by antigen (hCG), selecting a hybridoma which produces an aimed antibody, and obtain the clonal antibody produced by the hybridoma. Please refer to Kohler and Milstein technique (Nature 256(1975)495-497).

Polyclonal antibody is obtainable in the usual method, that is, immunizing an antibody producing animal (e.g. human, mouse, rat, rabbit, goat, horse) with an antigen (hCG), and separating the aimed antibody from the antiserum obtained from the animal.

An hCG is a glycoprotein of 36.7 kDa consisting of 237 amino acids. An hCG has an alpha-subunit and a beta-subunit. An alpha-subunit is same as luteinizing hormone (LH), follicle-stimulating hormone (FSH), and thyroid stimulating hormone, whereas a beta-subunit is a specific matter attributable to hCG. The reagent ingredient (the first reagent) used in the detection part (4) and the reagent ingredient (the second reagent) used as the labeling reagent can be either an anti hCG alpha antibody or an anti hCG beta antibody, each having a reaction bonding site at alpha-subunit or beta-subunit, but when anti hCG alpha antibody is used for one of the these ingredients anti hCG beta antibody should be used for the other one of the ingredients. As the reagent ingredient (the second reagent) used as the labeling reagent, it is preferable to use an anti hCG beta antibody, which has a reaction bonding site at the beta-subunit, from the viewpoint of reaction efficiency. As the reagent ingredient (the first reagent) used at the detection part (4), it is more preferably to use an anti hCG alpha antibody, which has a reaction bonding site at the alpha-subunit. As stated above, luteinizing hormone, follicle-stimulating hormone, and thyroid stimulating hormone have an alpha-subunit which is same as hCG's, and their structures as the antigen are similar to the structure of hCG so that in an immunochromatography system in which any of these is the antigen, a similar result is obtained if the detection system of the present invention is applied.

The following is the principle of the decision making:
1. A predetermined amount (normally 0.1 - 2 ml) of a sample (for example a woman's urine) is dripped onto a sample pad (1). When the sample is dripped, the reagent composition for immunochromatography retained or held in the sample pad (1) is dissolved in the moisture of the sample, and begins to immigrate with the sample.
2. The sample in which the reagent composition f immunochromatography is dissolved reaches the labeled material retained part (2) first. As the sample passes this part, the labeling reagent (the second reagent) retained by the labeled material retained part (2) is dissolved in the moisture of the sample and immigrate with the sample.
3. Then the labeling reagent dissolved in the moisture of the sample passes the detection part (4) on the chromatography medium (3). Here, the nonspecific reactions are suppressed by the reagent composition dissolved in the sample, and by virtue of the specific bonding reaction between the antigen and the antibody the hCG in the urine undergoes a specific reaction to combine with the antibody, which is held or fixed by retaining at the detection part (4), and with the labeling reagent in a manner such that the hCG in the urine is sandwiched between the antibody and the labeling reagent, whereby coloring of the detection part (4) takes place, if the woman is pregnant. If the woman is not pregnant, the labeling reagent dissolved in the moisture of the sample does not trigger a specific reaction as it passes through the detection part (4) on the chromatography medium (3), for the reason that no hCG is contained in the urine, so that there occurs no coloring at the detection part (4).
4. In the end, the moisture of the sample immigrates toward the absorption part (5).

In this manner, that is, by inspecting whether or not hCG exists in the urine, for example, it is possible to determine whether or not pregnancy is the case with precision.

It is possible to conduct a detection analysis similar to the one described above, by mixing the sample and the reagent composition for immunochromatography beforehand and then dripping and supplying the mixture to the sample pad (1) as the developing solution, instead of immersing or applying the reagent composition for immunochromatography to the sample pad (1) and then drying it to be retained or held there; the result obtained will be as precise as in the case of the above-described procedure. Also, if the sample is nasal mucus, phlegm, or rinse fluid from nasal cavity or pharynx, and if the detection target substance is influenza virus, then the reagent composition for immunochromatography is used as the sample thinner, and an anti-influenza virus monoclonal antibody is used as the reagent ingredient (the first reagent) set at the detection part (4) and also as the reagent ingredient (the second ingredient) used as the labeling reagent, and the collected sample is diluted to about 100 times thinner with the reagent composition for immunochromatography, and 150 micro liters of it is dripped/supplied to the sample pad (1) for development, and thereby it is possible to conduct a similar detection analysis as the above-described procedure with a result of similar precision.

### Examples

We will now explain the effectiveness of the present invention by giving examples, but the present invention shall not b construed to be limited by them.

### 1. Making of a decision part on the chromatography medium

An anti-hCG polyclonal antibody (alpha-hCGalpha) having a concentration of 1.0 mg/ml and diluted by phosphate buffer solution (pH 7.4) containing 5 wt% isopropyl alcohol was applied to a cellulose film (HF120, a product name, manufactured by Millipore Corporation) measuring 25 x 2.5 cm with an antigen coating machine (manufactured by BioDot Corporation), and was dried to complete the making of a decision part on the chromatography medium.

### 2. Preparation of labeling reagent solution

0.1 ml of phosphate buffer solution (pH 7.4) was added to and mixed with 0.5 ml of a gold colloidal suspension (manufactured by Tanaka Kikinzoku Kogyo: 80 nm), and to this was added 0.1 ml of anti-hCG monoclonal antibody (alpha-hCGbeta), which had been diluted with phosphate buffer solution, and the mixture was let to sit for ten minutes at room temperature. Next, to this was added 0.1 ml of 10 wt% bovine serum albumin (BSA) diluted with phosphate buffer solution, and the mixture was fully stirred, and then was subjected to centrifugal separation for 15 minutes at 8000 x g. The supernatant was removed and phosphate buffer solution was added, and the solution was well dispersed by means of an ultrasonic crusher to complete the preparation of a labeling reagent solution.

### 3. Preparation of chromatography medium

300 microliters of the prepared labeling reagent solution was uniformly applied to a glass fiber pad (manufactured by Millipore Corporation) measuring 16 x 100 mm, and the pad was dried in a vacuum drying machine, and thus made the labeling reagent retaining part. Next, onto a base consisting of a backing sheet (6) were pasted the nitrocellulose film, which makes the decision part, the labeling reagent retainer part, the sample pad made of glass fiber for receiving sample, and the absorption pad for absorbing the developed sample, the labeling reagent, etc. In the final step, the backing sheet was cut to have a width of 5 mm by a cutting machine, and thus the chromatography medium was made.

### 4. Measurement

Using the chromatography medium prepared in the manner stated in the paragraph 3 above, a measurement was conducted upon a 150-microliter sample diluted with phosphate buffer solution, which contained an hCG, the detection target substance, in a concentration of either 0 mIU/mL or 50 mIU/mL, to determine the existence or non-existence of the target substance. On this occasion of the test, the sample pad was let to receive and was immersed with tris-HCl buffer solution (pH 8.0) in a manner such that the latter accounts for 50 mM in relation to the diluted tested sample, was immersed with polyoxyethylene/polyoxypropyrene-alkyl ether (Nonion (a registered trademark) MN811 manufactured by Nippon Oil & Fats Co., Ltd.; the number of carbon atoms in the alkyl group = 14; [the molar ratio of oxypropylene]/[the molar ratio of oxyethylene] = 1.1) in a manner such that the latter accounts for 0.15 wt% in relation to the sample, and was immersed with EDTA in a manner such that the latter accounts for 0.83 mM, and then the pad was dried, whereafter the effect of the immersion of the sample pad with the above-named chemicals was estimated. visual judgment was conducted five minutes after the sample dripping, and if a red line of the test line at the decision part was observed, a rating represented by "+" was given, and if the red line was clearer, "++" was given, and if the red line was not observed, such a result was represented by "-".

The result was as follows: in the case wherein the sample pad was immersed neither with tris-HCl buffer, Nonion MN811, nor EDTA, there was observed a false positive sign attributable to a nonspecific reaction, and when a pad subjected to the immersion treatment was used no false positive sign was observed.

The results of the measurement of the effects caused by the immersion treatment upon the sample pads are shown in Table 1.

**effects caused by immersion treatment in sample pads**

| | | | | |
|---|---|---|---|---|
| Tris -HCI | - | | + | |
| MN811 | - | | + | |
| EDTA | - | | + | |
| | | | | |
| hCG (ng/mL) | 0 | 50 | 0 | 50 |
| visual observation | - | ++ | - | + |

### 5. Estimation of the effects attributable to the ratio of each composition

Based on the fact that the false positive sign is prevented from showing up by the addition of tris-HCl buffer, Nonion MN811, and EDTA to the sample pad, the ratio of each ingredient (to the diluted sample liquid) was examined.

On this occasion, the sample pad was let to receive and was immersed with tris-HCl buffer solution (pH 8.0) in a manner such that the latter accounts for 33 - 50 mM in relation to the diluted tested sample, and was immersed with polyoxyethylene/polyoxypropyrene-alkyl ether (Nonion (a registered trademark) MN811 manufactured by Nippon Oil & Fats Co., Ltd.; the number of carbon atoms in the alkyl group = 14; [the molar ratio of oxypropylene]/[the molar ratio of oxyethylene] = 1.1) in a manner such that the latter accounts for 0 - 0.3 wt% in relation to the sample, and was immersed with EDTA in a manner such that the latter accounts for 0.56 - 1.6 mM, and then the pad was dried, whereafter the effect of the immersion of the sample pad with the above-named chemicals was estimated.

Visual judgment was conducted five minutes after the sample dripping, and if a red line of the test line at the decision part was observed, a rating represented by "+" was given, and if the red line was clearer, "++" was given, and if the red line was not observed, such a result was represented by "-".

A PBS wherein the hCG is diluted to a concentration of 0 mIU/mL or 50 mIU/mL was used as the analyzed sample, and 150 micro liters was dripped for each test. visual inspection was conducted every five minutes, and when the red line of the test line was observed at the decision part a rating of "+" was given, and when the red line was observed only thinly a rating of "±" was given, and when the red line was not observed, a rating of "-" was given.

The proportionate concentration of each ingredient and the corresponding result of the visual inspection are indicated in Table 2.

Incidentally, in the case of 25 mIU/mL, if the rating of "+" was obtained after five minutes, no later judgment was conducted.

Herein below, we will explain the test examples of the present invention, but these test examples do not limit the scope of the invention.

### Test example 1

To 150 microliters of a diluted analyzed sample were added tris-HCl buffer (pH 8.0) in an amount of 33 mM, MN811 0.1 %, and EDTA 0.56 mM, and the sample pad was immersed with this mixture and was dried, and estimation was made. The result is given in Table 2.

### Test example 2

Except that MN811 was replaced by Tween20 in an amount of 0.1 %, the same procedure was taken as in the above-described Test example 1 and the sample pad was immersed with the resulting mixture and dried, and estimation was made. The result is shown in Table 2.

### Test example 3

To 150 microliters of a diluted analyzed sample were added tris-HCl buffer (pH 8.0) in an amount of 50 mM, MN811 0.15 %, and EDTA 0.83 mM, and the sample pad was immersed with this mixture and was dried, and estimation was made. The result is given in Table 2.

### Test example 4

To 150 microliters of a diluted analyzed sample were added tris-HCl buffer (pH 8.0) in an amount of 33 mM, MN811 0.05 %, Tween20 0.05 %, and EDTA 0.56 mM, and the sample pad was immersed with this mixture and was dried, and estimation was made. The result is given in Table 2.

### Test example 5

To 150 microliters of a diluted analyzed sample were added tris-HCl buffer (pH 8.0) in an amount of 33 mM, MN811 0.3 %, and EDTA 1.6 mM, and the sample pad was immersed with this mixture and was dried, and estimation was made. The result is given in Table 2.

### Test example 6

Similarly added were tris-HCl buffer (pH 8.0) in an amount of 100 mM, MN811 0.3 %, and EDTA 1.6 mM, and the result of the test on this are given in Table 2.

### Test example 7

Similarly added were tris-HCl buffer (pH 8.0) in an amount of 167 mM, Tween20 0.10%, MN811 0.20 %, and EDTA 1.6 mM, and the result of the test on this is given in Table 2.

### Test example 8

55 mM of 2-[4-(2-hydroxyl ethyl)-1-piperazinyl]ethane sulfonic acid (HEPES buffer solution) was used in place of the tris-HCl buffer of Test example 7, and the similar inspection was conducted and the result is given in Table 2. A false positive sign was suppressed during the first five minutes, but in the case of HEPES in a concentration of only 55 mM, the false positive sign was observed as the time passed.

**Table 2**

| effects corresponding to proportionate concentrations of ingredients in sample pad | | | | | | | | | | | | | | | | |
|---|---|---|---|---|---|---|---|---|---|---|---|---|---|---|---|---|
| | Test example1 | | Test example2 | | Test example3 | | Test example4 | | Test example5 | | Test example6 | | Test example7 | | Test example8 | |
| Tris-HCl | 33mM | | 33mM | | 50mM | | 33mM | | 33mM | | 100mM | | 167mM | | 55mM HEPES | |
| Tween20 | 0% | | 0.10% | | 0% | | 0.05% | | 0.00% | | 0% | | 0.10% | | 0.10% | |
| MN811 | 0.10% | | 0% | | 0.15% | | 0.05% | | 0.30% | | 0.30% | | 0.20% | | 0.20% | |
| EDTA | 0.56mM | | 0.56mM | | 0.83mM | | 0.56mM | | 1.6mM | | 1.6mM | | 1.6mM | | 1.6mM | |
| | | | | | | | | | | | | | | | | |
| hCG(ng/mL) | 0 | 25 | 0 | 25 | 0 | 25 | 0 | 25 | 0 | 25 | 0 | 25 | 0 | 25 | 0 | 25 |
| 5minutes | - | + | - | + | - | + | ± | + | - | + | - | + | - | + | - | + |
| 10minutes | - | | - | | - | | ± | | - | | - | | - | | ± | |
| 15minutes | ± | | ± | | - | | ± | | - | | - | | - | | ± | |

Even when Tween20 in Test example 2 was replaced by TritonX-100 or by a mixture of Tween20 and TritonX-100, the results were the same as in the case of using Tween20 only. Also, even when HEPES buffer solution in Test example 8 was replaced by phosphate buffer solution (pH 7.4), the result was the same as in the case of using HEPES buffer solution.

### 6. Estimation with regard to urine specimen, which tends to trigger nonspecific reaction more frequently

It had become clear that adding the above three elements to the sample pad is effective in suppressing the false positive sign. So a study was made to investigate whether ingredients in urine are effective in suppressing the false positive sign. Various elements are contained in urine, and nonspecific reaction is easy to be caused. In order to confirm the effectiveness in suppressing the false positive sign, samples were prepared by diluting the hCG in urine to 0 mIU/mL and 25 mIU/mL, respectively, and 150 micro liters of each was dripped in each test. Visual inspection was conducted every five minutes, and when the red line of the test line was observed at the decision part a rating of "+" was given, and when the red line was observed only thinly a rating of "±" was given, and when the red line was not observed, a rating of "-" was given.

The proportionate concentration of each ingredient and the result of the visual inspection are indicated in Table 3.

Incidentally, in the case of 25 mIU/mL, if the rating of "+" was obtained after five minutes, no later judgment was conducted.

As the result of the tests, it was confirmed that when the sample pad had not been treated a false positive sign was observed, but that when the sample pad had been treated with tris-HCl buffer, Nonion MN811 and EDTA the occurrence of false positive sign was suppressed. Also it was found to be the case that the higher the concentration of tris-HCl buffer solution, the longer the false positive sign is kept suppressed.

**Table 3**

| results of estimation upon urine specimens | | | | | | |
|---|---|---|---|---|---|---|
| Tris-HCl | - | | 33mM | | 100mM | |
| MN811 | - | | 0.3% | | 0.3% | |
| EDTA | - | | 1.6mM | | 1.6mM | |
| | | | | | | |

| hCG(ng/mL) | 0 | 25 | 0 | 25 | 0 | 25 |
|---|---|---|---|---|---|---|
| 5minutes | ± | + | - | + | - | + |
| 10minutes | + | | ± | | - | |
| 15minutes | + | | ± | | - | |
| 20minutes | + | | ± | | - | |
| 25minutes | + | | +(week) | | - | |

It was found through the above-described examination that by immersing a sample pad beforehand with nonionic surfactants such as tris-HCl buffer, EDTA and polyoxyethylene/polyoxypropylene block copolymer having an alkyl group at one end, the nonspecific reaction can be prevented.

By way of a different example, the same procedure was taken as in the preceding example except that the Nonion MN811 was replaced by Nonion TA-411 (a mixture manufactured by Nippon Oil & Fats Co., Ltd.; the number of carbon atoms in the alkyl group = 11 through 18; [the molar ratio of oxypropylene]/[the molar ratio of oxyethylene] = 0.15). It was possible to achieve a similar goal as was done and shown in Table 1, Table 2 and Table 3.

By way of still another example, the same procedure was taken as in the said preceding example except that the Nonion MN811 was replaced by ADEKA TOL LB-53B (manufactured by ADEKA CORPORATION: number of carbon atoms in alkyl group = 12, [the molar ratio of oxypropylene]/[the molar ratio of oxyethylene] = 0.3). It was possible to achieve a similar goal as was done and shown in Table 1, Table 2 and Table 3.

By way of a still different example, the same procedure was taken as in the said preceding example except that the EDTA was replaced by nitrilotriacetic acid (NTA). When NTA was used, although there occurred some variation in effectiveness among the individual matters used, it was confirmed that it was possible to achieve a similar goal as was done and shown in Table 1, Table 2 and Table 3.

Other embodiments were tried adopting, for example, the following combinations, and the similar goal was achieved.

| | buffer solution | nonionic surfactant | chelating agent |
|---|---|---|---|
| (1) | acetate buffer solution | MN81 | NTA |
| (2) | phosphate buffer solution | ADEKA TOL LB-53B | EDTA |
| (3) | tris-HCl buffer solution | Nonion TA-411 | ASDA |

The detective kit of the present invention is capable of suppressing nonspecific reactions and thus capable of detecting hCG in urine specifically, so that it enables prompt and simple detection of hCG to achieve pregnancy test, and thus is industrially useful and applicable.

## Claims

1. A reagent composition for immunochromatography for detecting from an analyzed sample a target object selected from a group consisting of human chorionic gonadotropin (hCG), luteinizing hormone (LH), follicle-stimulating hormone (FSH) and thyroid stimulating hormone (TSH), said reagent composition being **characterized by** containing a buffer solution, a chelating agent, and a non-ionic surfactant made of a polyoxyethylene/polyoxypropylene block copolymer of which the molar ratio of the oxyethylene repetition units in the polyoxyethylene block having an alkyl group at one end to the oxypropylene repetition units in the polyoxypropylene block is in a range of 0.1 - 1.5.

2. The reagent composition for immunochromatography as claimed in claim 1 wherein said chelating agent is one based on aminocarboxylic acid.

3. A diluted reagent composition for immunochromatography for detecting from an analyzed sample a target object selected from a group consisting of human chorionic gonadotropin (hCG), luteinizing hormone (LH), follicle-stimulating hormone (FSH) and thyroid stimulating hormone (TSH), the reagent composition being **characterized by** containing a buffer solution, a chelating agent, and a non-ionic surfactant made of a polyoxyethylene/polyoxypropylene block copolymer of which the molar ratio of the oxyethylene repetition units in the polyoxyethylene block having an alkyl group at one end to the oxypropylene repetition units in the polyoxypropylene block is in a range of 0.1 - 1.5.

4. An immunochromatography device **characterized by** comprising substantially and sequentially of a sample introduction part, a marker holding part, a chromatography medium part, a detection part for detecting from an analyzed sample a target object selected from a group consisting of human chorionic gonadotropin (hCG), luteinizing hormone (LH), follicle-stimulating hormone (FSH) and thyroid stimulating hormone (TSH), and an absorption part, and also **characterized by** having, at a location between an end of the sample introduction part and the absorption part, a site which includes a reagent composition for immunochromatography containing a buffer solution, a chelating agent, and a non-ionic surfactant made of a polyoxyethylene/polyoxypropylene block copolymer of which the molar ratio of the oxyethylene repetition units in the polyoxyethylene block having an alkyl group at one end to the oxypropylene repetition units in the polyoxypropylene block is in a range of 0.1 - 1.5.

5. The immunochromatography device as claimed in claim 4 wherein the chelating agent is one based on aminocarboxylic acid.

6. A detective kit **characterized by** including an immunochromatography device, which comprises substantially and sequentially of a sample introduction part, a marker holding part, a chromatography medium part, a detection part for detecting from an analyzed sample a target object selected from a group consisting of human chorionic gonadotropin (hCG), luteinizing hormone (LH), follicle-stimulating hormone (FSH) and thyroid stimulating hormone (TSH), and an absorption part, and also **characterized by** having, at a location between an end of the sample introduction part and the absorption part, a site which includes a reagent composition for immunochromatography containing a buffer solution, a chelating agent, and a non-ionic surfactant made of a polyoxyethylene/polyoxypropylene block copolymer of which the molar ratio of the oxyethylene repetition units in the polyoxyethylene block having an alkyl group at one end to the oxypropylene repetition units in the polyoxypropylene block is in a range of 0.1 - 1.5.

7. A developing solution for immunochromatography used for detecting from an analyzed sample a target object selected from a group consisting of human chorionic gonadotropin (hCG), luteinizing hormone (LH), follicle-stimulating hormone (FSH) and thyroid stimulating hormone (TSH), said developing solution being **characterized by** containing a buffer solution, a chelating agent, and a non-ionic surfactant made of a polyoxyethylene/polyoxypropylene block copolymer of which the molar ratio of the oxyethylene repetition units in the polyoxyethylene block having an alkyl group at one end to the oxypropylene repetition units in the polyoxypropylene block is in a range of 0.1 - 1.5.

8. An immunochromatography **characterized by** using, as its developing solution for constituting a mobile phase, a developing solution for immunochromatography which contains a buffer solution, a chelating agent, and a non-ionic surfactant made of a polyoxyethylene/polyoxypropylene block copolymer of which the molar ratio of the oxyethylene repetition units in the polyoxyethylene block having an alkyl group at one end to the oxypropylene repetition units in the polyoxypropylene block is in a range of 0.1 - 1.5, to thereby detect from an analyzed sample a target object selected from a group consisting of human chorionic gonadotropin (hCG), luteinizing hormone (LH), follicle-stimulating hormone (FSH) and thyroid stimulating hormone (TSH).
